# EUROPEAN PATENT APPLICATION

(11) **EP 2 011 460 A1**
(43) Date of publication of application: **07.01.2009**
(21) Application number: 07111972.1
(22) Date of filing: 06.07.2007
(51) Int. Cl.: A61F 13/15

(54) **Pantiliner**

(71) Applicant: The Procter and Gamble Company, Cincinnati, Ohio 45202 (US)
(72) Inventor: D'Addario, Roberto, 65019, Pianella (IT); Gagliardi, Ivano, 65123, Pescara (IT)
(74) Representative: L'Huillier, Florent Charles

(57) **Abstract**

A pantiliner 10 comprising a nonwoven topsheet 26, a nonwoven backsheet 22 and an absorbent core 20 disposed between said topsheet and said backsheet. A fluid impermeable barrier 24 is disposed between the absorbent core and the backsheet. The fluid impermeable barrier comprises a coloured pattern 34 visible through at least one of the topsheet or the backsheet. The area of the pantiliner outside the fluid impermeable barrier is a breathable zone 32 representing at least 20% of the overall surface of the pantiliner.

## Description

### FIELD OF THE INVENTION

This invention relates to feminine hygiene articles, in particular pantiliners.

### BACKGROUND OF THE INVENTION

Pantiliners are relatively thin feminine hygiene articles designed to protect the user's underwear and clothing from soiling by vaginal discharges during or outside the menstrual period. Many women have developed the habit of wearing an absorbent article between their menstrual periods to protect their clothing from any vaginal discharges, including light urinary discharge, and sometimes anal discharge. Because a sanitary napkin is generally too bulky for constant wear, such user's generally utilize pantiliners.

Most pantiliners have a non-breathable backing layer, commonly referred to as backsheet. The backsheet of typical pantiliners can for example be a fluid impermeable polymer film. A fluid impermeable layer prevents fluids absorbed into the article from wetting through to the user's underwear. However, a fluid impermeable backsheet may also make the pantiliner hot and uncomfortable, due to trapped moisture in the absorbent core.

U.S. Pat. No. 4,059,114 issued to Richards on Nov. 22, 1977 discloses a disposable garment shield having a moisture barrier ply constructed of a blown microfiber web which is fluid impermeable but vapor permeable. Also, U.S. Pat. No. 4,681,587 issued to Anderson et al. on July 21, 1987 discloses an absorbent article such as a pantiliner provided with at least one ventilation area which allows the passage of vapor to provide cooling and drying effects so that the pantiliner is more comfortable to wear. WO2004/006818 discloses absorbent articles having a graphic visible through the body contacting surface.

WO2005/084597 discloses a pantiliner comprising a fluid permeable topsheet, a fluid permeable backsheet, and an absorbent core disposed in-between. The topsheet and the backsheet comprise relatively hydrophobic nonwoven material and define an article periphery that is substantially larger than the core periphery. The area between the core periphery and the article periphery is a breathable zone. The article further comprises a fluid impermeable barrier between the backsheet and the absorbent core, the fluid impermeable barrier being disposed within the core periphery.

While the feminine hygiene articles disclosed in WO2005/084597 provide good performance in terms of breathability and protection, the inventors have found that because all the layers of the article, including the fluid impermeable barrier layer, are substantially white, the presence of the barrier layer situated below the core is not perceptible by the user. When the backsheet and topsheet are made of nonwoven materials, which are normally relatively fluid permeable, a prospective user may therefore not realize that the article has an improved protection against leaks, because the fluid impermeable barrier material is not visible. The overall size and placement of the core itself may also be difficult to determine by the user. This may induce the user to change the pantiliner prematurely or even discourage a prospective user from using such a pantiliner. The inventors have come to the insight that there was a yet unrecognized need to make the fluid impermeable barrier material of such pantiliners more visible.

### SUMMARY OF THE INVENTION

The invention relates to a pantiliner comprising a nonwoven topsheet, a nonwoven backsheet and an absorbent core disposed between said topsheet and said backsheet. The topsheet and backsheet are joined and define the overall surface of the pantiliner. The pantiliner comprises a fluid impermeable barrier layer disposed between the absorbent core and the backsheet. The absorbent core defines a core periphery. The fluid impermeable barrier comprises a coloured pattern visible through at least one of the topsheet or the backsheet. The area of the pantiliner outside the fluid impermeable barrier is a breathable zone, said breathable zone representing at least 20% of the overall surface of the pantiliner. The fluid impermeable barrier layer may advantageously not extend beyond the core periphery, but this is not required.

### BRIEF DESCRIPTION OF THE DRAWINGS

FIG. 1 is a partially cut away perspective view of a pantiliner of the present invention having a generally oval shaped absorbent core.
FIG. 2A is a cross-sectional view of the cross section 2-2 in one embodiment of a pantiliner as shown in FIG. 1.
FIG. 2B is a cross-sectional view of the cross section 2-2 in another embodiment of a pantiliner as shown in FIG. 1.
FIG. 3 is a partially cut away perspective view of another pantiliner of the present invention with a differently shaped core.
FIG. 4 is a partially cut away perspective view of another pantiliner of the present invention wherein the backsheet is pigmented.
FIG. 5 is a partially cut away perspective view of another pantiliner of the present invention wherein the backsheet is pigmented.
FIG. 6 is a perspective view from below of the pantiliner of Fig.4.

### DETAILED DESCRIPTION OF THE INVENTION

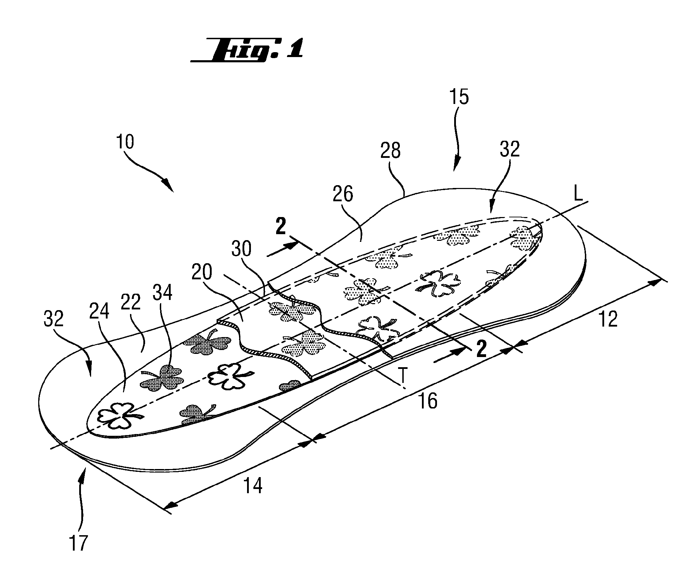

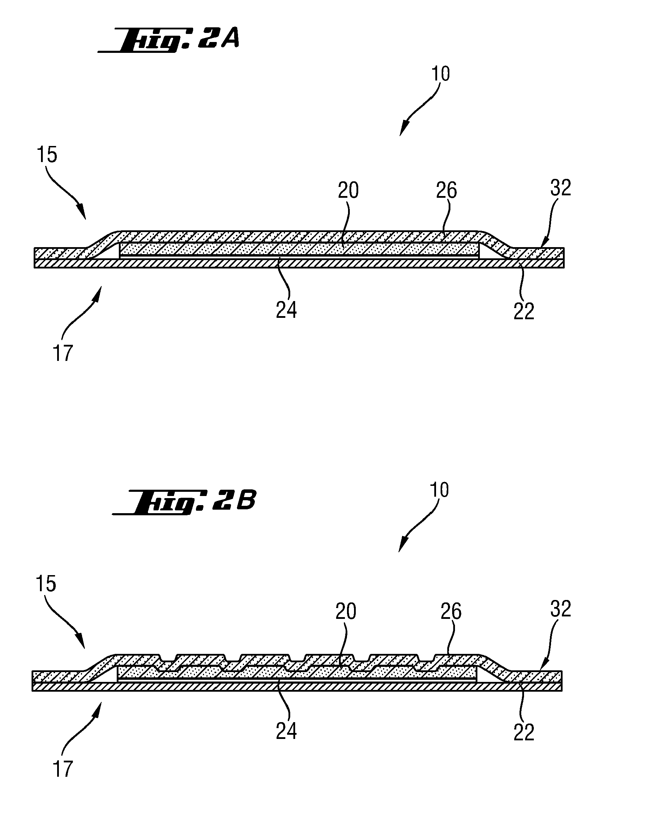

A first exemplary embodiment of a pantiliner 10 according to the invention is shown in partially cut-away perspective view in Fig. 1 and in two alternative cross-section represented in Figs. 2A and 2B. The pantiliner 10 has two end regions 12 and 14 and a middle region 16. The pantiliner 10 has a body-facing side 15 and a garment facing side 17.

From top to bottom, the exemplary pantiliner 10 represented comprises the following layers: a topsheet 26, an absorbent core 20, a fluid impermeable barrier layer 24 and a backsheet 22. The "top" of article is defined herein as the body-facing side 15 when the liner is in use, the "bottom" is the opposite surface of the article, i.e. the garment facing surface 17.

While the pantiliner 10 may have any shape known in the art, a typical shape is generally "hourglass" shaped, tapering inwardly from a relatively greater transverse width in a portion of one of the end regions to a relatively smaller transverse width at the middle region. Transverse width is generally defined as the dimension perpendicular to the dimension, which is defined as length, running from end region 12 to end region 14 parallel to longitudinal centerline L.

The garment facing side 17 can have thereon pressure sensitive adhesive for affixing to a wearer's undergarments. Typically, such adhesive is covered with a release strip which is removed before affixing to the undergarment.

Pantiliners can also be provided with lateral extensions known commonly in the art as "flaps" or "wings" (not shown) intended to extend over and cover the panty elastics in the crotch region of the user's undergarment. However wings are normally not used with pantiliners.

Many commercially proposed pantiliners have a substantially overall white appearance. The inventors have surprisingly found that overall whiteness may make it more difficult for the user to discern the overall size and placement of the core, particularly in the case of articles having an absorbent core with a surface substantially smaller than the overall surface area of the pantiliner, and/or when the pantiliner is relatively thin. This may discourage some users from using relatively thin pantiliners because of difficulties to assess when the absorbed fluid is approaching the side of the core and/or when the article should be changed to prevent side leakage. This is particularly a problem for relatively thin article having a thickness of less than about 4 mm. The inventors have also surprisingly found that another problem existed when the backsheet and/or topsheet are made of nonwoven materials. Nonwovens are normally not fluid impermeable and if the user does not recognize the presence of the fluid barrier layer, this may cause an unfounded lack of trust in the leak-preventing ability of the product.

The inventors have come to the insight that a cost-effective solution to these yet unrecognized problems was to provide the fluid impermeable barrier 24 with a coloured pattern 34 visible through at least one of the topsheet 26 (which normally at least partially forms the body-facing side 15) or the backsheet 22 (which normally at least partially constitutes the garment-facing side 17) or both, thereby providing several benefits.

A first benefit is that, as indicated above, the coloured pattern 34 may highlight or materialize to the user the presence of the protective layer provided by the fluid impermeable barrier layer, which would otherwise be invisible. A second benefit is that the coloured pattern may improve the appearance of the article without direct contact of the coloured layer with either the skin of the user or the user's garment because it is placed between topsheet and backsheet. Some users dislike having a coloured layer in direct contact with their skin or their undergarment. A third benefit, in particular when the fluid impermeable barrier layer is contiguous with the core, is that the position and size of the core is outlined, which may otherwise be difficult to recognize, in particular for relatively thin pantiliners.

The coloured pattern 34 is visible by transparency through the body-facing side 15 and/or the garment-facing side 17 of the article 10. By "visible", it is meant that a subject having a good vision in both eyes (10/10) holding the article at a distance of about 50 cm in a brightly lit room with incandescent light can see the printed pattern 34. Although cores in general may be relatively opaque, they are normally sufficiently thin and transparent in the case of a pantiliner to allow the printed fluid impermeable layer 34 underneath to be seen through the core and topsheet.

As used herein, the term "pattern" generally refers to any shapes, forms, graphics, symbols and combinations thereof. Examples of pattern can be purely abstract geometric shapes, such as circles or waves, and/or be evocative of natural elements such as floral patterns, and/or be evocative of certain texture or fabrics such as laces. "Pattern" also includes an uniformly coloured surface. By "coloured" we mean non-white.

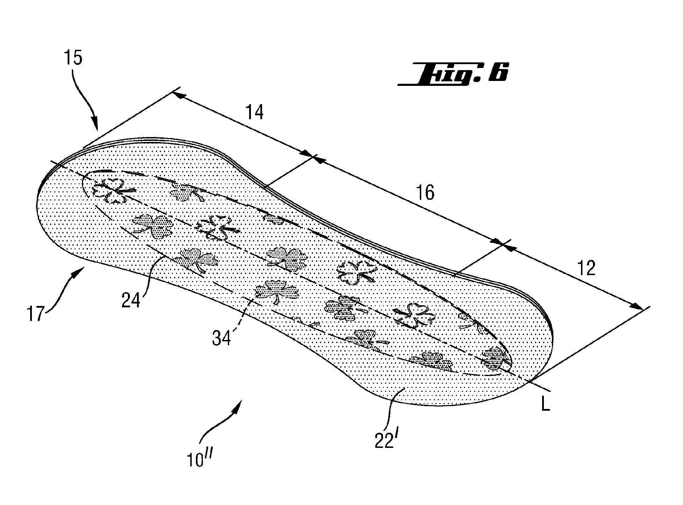

An efficient way to provide a barrier layer 24 having a coloured pattern 34 is by printing one of the surface of the barrier layer 24 with the coloured pattern. Conventional printing methods that may be used include flexo printing and roto gravure printing. The side of the barrier layer 24 that is printed may be the garment-facing side, the body-facing side, or both. The barrier layer 24 may be sufficiently transparent so that it is not critical on which side of the barrier material the coloured pattern is printed. However, it may be advantageous to print on the bodyfacing side of the barrier layer 24 if the core 20 is somewhat opaque, to ensure a better visibility of the printed pattern 34 through the topsheet 26 and the core 20. The coloured pattern 34 should be printed with an ink sufficiently strong to be at least partially visible through the topsheet, or the backsheet or both. The coloured pattern 34 represented in the Figures is visible through both topsheet and backsheet, as exemplary represented on Fig. 1 and Fig. 6. The colour used may be of any shade, for example black, pink, violet, green, purple, blue or yellow, or even a combination of different coloured inks.

The coloured pattern 34 may comprise, as represented in Fig. 1, a series of dispersed, discrete printed elements which may be identical or differ within the printed pattern 34. Of course, other discrete elements than those represented may be used, for example floral decorative elements (flowers, leaves), stars, or any other decorative elements. The coloured pattern 34 may also consist of a continuous element rather than discrete elements.

If it is desired that the barrier layer 24 is uniformly coloured, a fluid impermeable barrier material which is inherently coloured may be used to form the barrier layer 24. For example, a pigmented polyolefin material, in which pigments have been directly incorporated in the polymer during the manufacture of the film.

The fluid impermeable barrier layer 24 (herein "barrier layer") is normally interposed between the absorbent core 20 and backsheet 22. One of the function of the barrier layer 24 is to prevent the fluids retained by the absorbent core 20 from striking through the garment-facing side 17 of the pantiliner 10 and soiling adjacent garments. The barrier layer 24 is normally not breathable, and area of the pantiliner outside the barrier layer normally constitutes a breathable zone 32.

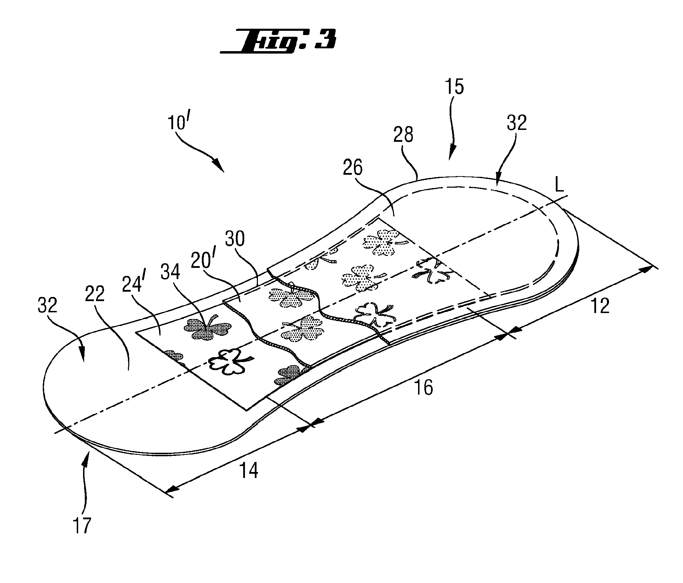

The barrier layer 24 may take various shapes and forms, provided that a sufficiently large breathable zone remains. The barrier layer 24 may advantageously not extend beyond the core periphery 30 of the pantiliner 10. In other words, the barrier layer 24 may be entirely situated under the absorbent core 20 and within the area defined by the periphery 30 of the absorbent core 20. As shown in Fig.1, for example, the barrier layer 24 may be contiguous with the core 20, so the barrier layer 24 and the core 20 have about the same surface area and placement, in which case the barrier layer 24 extends to but does not exceed the core periphery 20. The barrier layer 24' may also have a smaller surface than the surface of the core, as shown on Fig. 3. This is particularly advantageous if the surface area of the core is relatively large, as exemplified in Fig. 3, because if the barrier layer 24' in that case was contiguous with the core 20' then the breathable zone may be relatively small and only provide limited breathability benefit. In that case, the barrier layer 24' is however advantageously present at least in the middle region 16 of the pantiliner. The middle region is relatively more exposed to vaginal discharges than the two end regions 12 and 14.

It is also not excluded that the barrier layer 24 may also extend beyond the core periphery 30, but the barrier layer 24 should not extend as to cover the whole of the article surface (and thus form a secondary backsheet) because then there would be no breathable zone 32.

The barrier layer 24 may be made of any suitable fluid impermeable materials that can prevent the fluid from striking through. Polyolefin films are normally suitable, in particular polyethylene (PE) film. These films may be relatively thin, for example of from about 10 gsm to about 40 gsm. An example of commercially available film with a printed leaves pattern is a Printed PE film 24gsm available from Daedong Co. LTD under the designation DNF0417CLeaves-PG476 - Thickness 0.06mm.

The barrier layer 24 may be directly in contact with the absorbent core 20 or may be separated from it by another layer. The barrier layer may for example be attached to the absorbent core with conventional adhesive means such as glue. If adhesive means are used, the whole of the barrier layer may be applied with the adhesive means or only a part of it.

The function of the absorbent core 20 is to absorb and store bodily fluids discharged. The absorbent core 20 can be made of any materials known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, a web of polymeric fibers, and a blend of polymeric fibers. The absorbent core 20 may be relatively hydrophilic. By "relatively hydrophilic" it is meant that the core 20 is hydrophilic with respect to the portion of the pantiliner 10 in the breathable zone 32, which may be relatively hydrophobic. Hydrophilicity can be determined by any number of means known in the art, including by reference to contact angles of fluid on a surface. However, relative hydrophilicity may be considered as a more relevant factor, such that surface tensions in the pantiliner/fluid system tend to drive fluid deposited on the body-surface of the pantiliner into the absorbent core 20.

The absorbent core 20 may be relatively thin, for example less than about 2 mm in thickness, or less than about 1 mm, or even less than about 0.5 mm in thickness. The absorbent core can comprise absorbent gelling materials (AGM), including AGM fibers. A specific example of absorbent core 20 may be an airlaid cellulose material having a basis weight between about 50 gsm to about 100 gsm. An exemplary absorbent core is a 63 gsm (60 gsm airlaid + 5 wt % AGM in fiber form) available from Concert GmbH, Germany, under the designation VH063.200.B001 Another specific example is a TBAL (thermal bonded air-laid) 80 gsm available from Nanning Qiaohong New Materials Co. LDT under the designation BJA080T. Exemplary cores may also be made of an airlaid carded, nonwoven material having a basis weight between about 50 gsm and about 100 gsm, for example an 80 gsm carded airlaid nonwoven web comprising 2.2 dtex hydrophilic polypropylene fibers and 10 dtex superabsorbent fiber, available from Sandier under the name Sawabond 24-00-32.

Absorbent core 20 is cut to a shape, the edges of which define a core periphery 30. The core periphery 30 may have any suitable shapes, such as rectangular, circular, oval, elliptical, hour-glass or the like. Absorbent core 20 can be generally centered within the pantiliner 10.

The pantiliners of the invention comprise a nonwoven topsheet 26 which normally at least partially forms the body-facing layer 15 of the pantiliner 10. The topsheet 26 may be a soft, smooth, compliant, porous nonwoven material which is comfortable against human skin and through which vaginal discharges can pass. The nonwoven topsheet 26 can comprise fibers as are known in the art, including bicomponent and shaped fibers. One example of suitable topsheet material is a relatively hydrophobic 20 gsm spunbonded nonwoven web comprising bicomponent fibers of the sheath core type (PP/PE) available from Pegas a.s., Czech Republic, under the designation 10XXN008005.

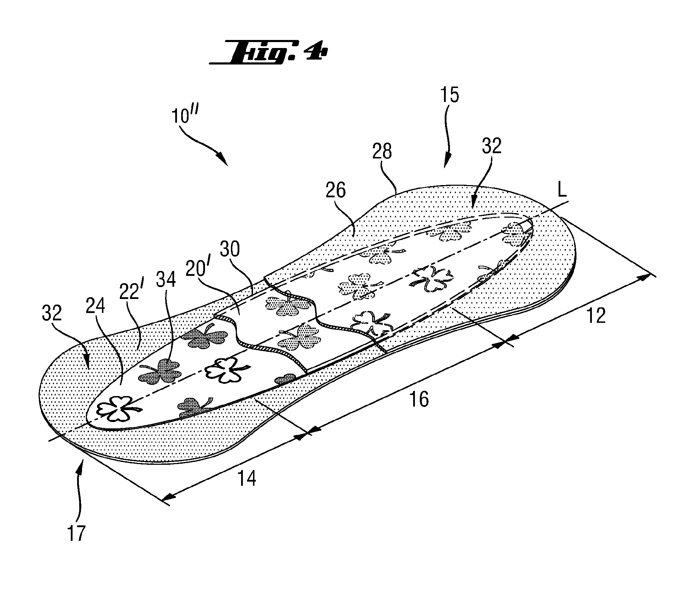

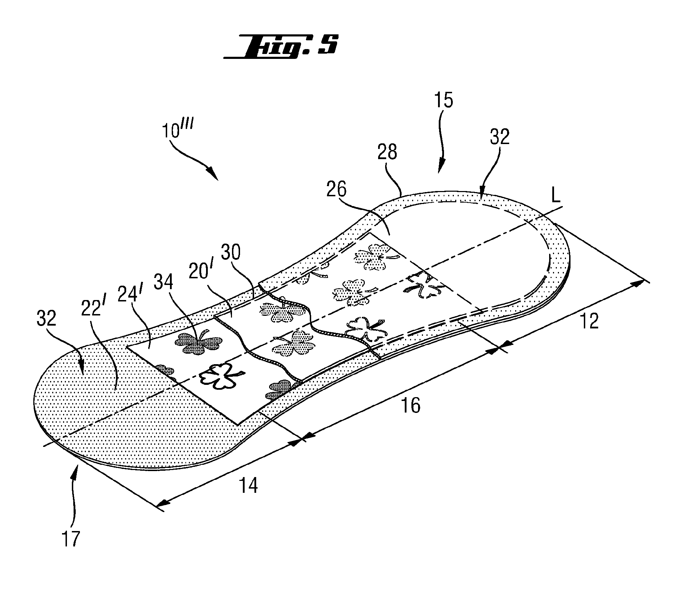

The backsheet 22 is the layer that normally at least partially forms the garment-facing 17 side of pantiliner 10. The backsheet 22 is made of a nonwoven material, which may provide a degree of softness and vapor permeability. Nonwoven webs are normally soft, smooth, compliant and vapor pervious material, and low noise producing so that movement does not cause unwanted sound. The backsheet 22 may be for example a nonwoven web having a basis weight between about 15 gsm and about 50 gsm, for example a relatively hydrophobic 23 gsm spunbonded nonwoven web of 4 denier polypropylene fibers available from Fiberweb Neuberger, under the designation F102301001. Although this is not required, the backsheet may be itself coloured to improve the appearance of the pantiliner and/or further improve the contrast between the fluid barrier layer and the rest of the article. For example a pigmented nonwoven material may be used to form the backsheet 22', as represented in the embodiments of Fig.4, 5 and 6. An example of pigmented green backsheet is Applegreen F1XXX66001 (where XXX stands for basis weight, for example 10 gsm to 50gsm) available from Fibertex Neuberger.

It may be advantageous that both topsheet 26 and backsheet 22 are hydrophobic, fluid permeable nonwoven webs. If hydrophobic, or rendered hydrophobic, such that a drop of fluid makes a contact angle of at least about 75 degrees with respect to the surface of the web, the nonwoven can be fluid repellent, thereby functioning as a fluid barrier although it is, in fact, fluid permeable.

At least one, and usually both, of topsheet 26 and backsheet 22 define a shape, the edge of which defines the periphery 28 of the pantiliner 10. As represented, both topsheet 26 and backsheet 22 may together define the pantiliner periphery 28. The two layers can be die cut, for example, after combining all the components into the structure of the pantiliner as described herein. The surface within the periphery 28 of the pantiliner 10 is herein referred to as the overall pantiliner surface.

The pantiliner periphery 28 may be substantially larger than the core periphery 30, and the barrier layer 24 be contiguous with the core periphery 30, as exemplary represented on Fig.1. In this manner, the region of pantiliner 10 between the core periphery 30 and the pantiliner periphery 28 defines a breathable zone 32, which may be comprised only of the topsheet and the backsheet 22. As represented in Fig. 1, the breathable zone 32 may extend laterally from the core periphery 30 to the pantiliner periphery 28. A minimum level of breathability is achieved by the use of porous nonwoven materials for the topsheet and/or backsheet. If the breathable zone comprises both the topsheet and the backsheet, it can also comprise means for joining the two, such as adhesive means, including hot melt adhesives. In such an embodiment the adhesive should preferably not be applied so as to render the topsheet and/or backsheet completely non-porous.

In general, the absorbent core 20 may be located symmetrically with respect to the longitudinal centerline L and transverse centerline T, but other placements are possible. For example, the core may be placed more toward one of either the first end region 12 or second end region 14. In this manner, more of the breathable zone 32 can be disposed over the anal region of the wearer, for example.

The breathable zone 32, which is normally the zone of the pantiliner 10 not covered by the barrier layer 24, represents at least about 20% of the overall surface area of the pantiliner. The breathable zone 32 may advantageously be larger than 20%, and represent at least about 25%, 30%, 40%, 50% or 60% of the overall surface area of the pantiliner. In the embodiment as represented in Fig. 1, the absorbent core 20 may have a surface area of about 35 cm² and the breathable zone has surface area of about 38 cm², for an overall pantiliner surface area of about 73 cm².

A benefit of having a relatively large breathable zone 32 is enabling more effective coverage of the wearer's undergarment without increasing overall bulk of the article. That is, by concentrating the absorbent core 20 to a relatively small central region of the pantiliner, bulk is reduced. By having a breathable region 32 surrounding the absorbent core 30, the garment-facing side 17 of the pantiliner backsheet 22 also has more surface area available for adhesive attachment to be adhered to the undergarment.

On the other hand, it may also be desirable to have a core 20' with a relatively larger surface area in the horizontal plane, for example to provide for a pantiliner 10' with a larger absorbing zone, a higher absorbency capacity or both. As represented in Fig. 3, the core periphery 30 may in that case extend almost to, or in some cases even directly to, the pantiliner periphery 28. When the core represents a relatively large percentage of the overall surface of the article, it may be desirable to provide for a fluid impermeable barrier layer 24' which is substantially smaller than the absorbent core 20'. As represented in Fig. 3, part of the area under the absorbent core in the two end regions 12 and 14 may be left without a fluid impermeable barrier to provide for a breathable zone 32 of sufficient size. Since most of the liquids are absorbed in the central region of the pantiliner, risk of leakage through one of the end regions 12, 14 is limited.

The dimensions of the pantiliners of the invention may range from relatively small to relatively large, including the usual sizes for pantiliners. Exemplary values for the length dimension of pantiliner 10 as measured parallel to the longitudinal axis L can be from about 6 cm (so called "micro" products), to about 25 cm (for so called "extralong" products). The greatest width dimension of pantiliner 10 as measured parallel to the transverse axis T may also vary, in general from at least about 3 cm to about 10 cm. Exemplary values for a generally hour-glass shaped pantiliner is a minimum width dimension of about 5 cm in the middle, and a maximum width dimension at the end regions of about 6.5 cm, and a length dimension of about 14 cm.

The minimum and maximum overall surface area of the pantiliner (i.e., the area of the pantiliner when viewed in flat, plan view) of the pantiliner 10 and absorbent core 20 is limited only by the intended use, including the relative size of a wearer's undergarments. Generally, the pantiliner overall surface area may be of at least about 60 cm², or at least about 75 cm², or at least about 90 cm², and can be at least about 100 cm². Likewise, the absorbent core 20 can cover an area of at least about 20 cm², or at least about 25 cm², or at least about 35 cm² and can be at least about 45 cm² or more.

The pantiliners of the invention are relatively thin feminine hygiene articles, in particular compared to bulkier products generally referred to as sanitary napkins. The pantiliners of the invention may have a thickness of less than about 5 mm, or less than about 4mm, or even less than about 3mm. One exemplary method for measuring thickness is provided further below.

All the components of the pantiliners can be adhered together using conventional means, including adhesives such as hot melt adhesives, as is known in the art. The adhesive can be Savare' PM 17, which may for example be applied using Dynafiber HTW system or Summit System Nordson. As mentioned above, the adhesive used in the breathable zone should preferably not render the breathable zone non-breathable, i.e., not render either the topsheet or backsheet non-porous. Other benefits of keeping the breathable zone porous include preventing the pantiliner from sticking to the skin of the wearer, thereby increasing discomfort.

The topsheet 26 and backsheet 22 advantageously have externally-facing surfaces that are hydrophobic, or rendered to be hydrophobic. By hydrophobic is meant that a drop of water placed on the surface does not readily wet out and into the nonwoven. In one embodiment, the hydrophobic body-facing surface is fluid repellent, such that a drop of water placed thereon remains on the surface for an extended period of time, for example 10 to 30 minutes.

However fluid deposited on the portion of the topsheet overlying the relatively hydrophilic absorbent core 20 is readily drawn through the topsheet and into the absorbent core. On the other hand, fluid deposited outside of the region overlying the absorbent core, which is normally within the breathable zone 32, does not get easily absorbed, and does not easily strike through to the garment facing side of pantiliner 10.

Therefore, in use, the pantiliner 10 of the present invention may provide for a very thin, flexible, comfortable pantiliner having a relatively small centrally-disposed hydrophilic "pocket" surrounded by a fluid repellent breathable zone 32. The relatively hydrophobic breathable zone 32 acts as an effective barrier to fluid movement out of the region of the absorbent core 20. Thus, in use, fluid discharged from the body can be quickly absorbed, and prevented from running off the pantiliner and onto the user's garments.

The breathable zone 32, which is normally the zone of the article not covered by the barrier layer 24, may completely surround the absorbent core 20. That is, in no portion of pantiliner 10 does the core periphery 30 coincide with pantiliner periphery 28, but the two peripheries may thus be always separated by a region of breathable zone 32. In this embodiment, the breathable zone 32 is a continuous band of breathable zone that completely encircles, or surrounds, absorbent core 20.

In order to promote faster fluid entry into the absorbent core 20 the topsheet 26 and absorbent core 20 can be processed so as to have a certain amount of fiber entanglement. Entanglement can be accomplished by mechanical means known in the art. For example, as shown in cross-section in Fig. 2B, the absorbent core 20 and the topsheet 26 can be entangled by embossing, such that fibers of the topsheet 26 are forced into fibers of absorbent core 20. Other means, including mechanical treatment means known in the art, such as what is commonly referred to as "ring rolling" can also be used to accomplish fiber entanglement. It is believed that fiber deformation-inducing treatments, such as embossing, not only helps expose deposited fluid to hydrophilic fibers in the absorbent core, it also forms small-scale "hills" and "valleys" that help contain deposited fluid on the pantiliner over the "pocket" of the hydrophilic absorbent core 20.

### THICKNESS MEASUREMENT

The following specific method may be used to measure the thickness of the pantiliners of the invention. However, pantiliners are generally relatively thin and not bulky, so that the measurement is relatively independent of the pressure applied for example compared with for bulky articles such as thick pads.

The equipment may comprise an apparatus capable of measuring thickness with a 0.01 mm tolerance. A commercial supplier of such equipment is for example Ono Sokki (www.onosokki.net), for example their Caliper Gauge GS-503 and digital readout DG 2610 may be used. The caliper gauge is fitted with a foot, which may have an exemplary 24.13 mm diameter. A suitable pressure exerted when the measurement is made is 0.689 kPa.

The test procedure is as follows. Make sure the micrometer is zeroed. Place the article without the release cover on the base plate, the topsheet facing up. If the article was provided in a compressed state (as is sometimes the case in certain packaging), the article is let to rest about 10 mn before its thickness is measured. Similarly, if the article was provided folded, the article is first opened and let about 10 mn to rest in its "flat" shape. Position the article on the base plate so that when the foot is lowered, it is in the center of the article. Let the foot gently lowers itself onto the article at a rate of 5 mm/sec +/- 2 mm/sec. Determine the article caliper by reading the micrometer dial 10 seconds after the foot comes to rest. The shaft and foot should deliver approximately 32 grams of force for a pressure of 0.69 +/- 0.02 kPa to the sample with the above mentioned foot having a diameter of 24.13mm.

## Claims

1. A pantiliner (10) comprising:
a. a nonwoven topsheet (26),
b. a nonwoven backsheet (22), wherein said topsheet (26) and backsheet (22) are joined and define the overall surface of the pantiliner,
c. an absorbent core (20) disposed between said topsheet (26) and said backsheet (22), wherein said absorbent core (20) defines a core periphery (30),
d. a fluid impermeable barrier layer (24) disposed between said absorbent core (20) and said backsheet (22), said fluid impermeable barrier layer (24) comprising a coloured pattern (34) visible through at least one of the topsheet (26) or the backsheet (22),
wherein the area of the pantiliner outside the fluid impermeable barrier layer (24) is a breathable zone (32), said breathable zone (32) representing at least 20% of the overall surface area of the pantiliner.

2. A pantiliner according to claim 1 wherein said fluid impermeable barrier layer (24) does not extend beyond the core periphery (30).

3. A pantiliner according to claim 2 wherein the fluid impermeable barrier layer (24) is contiguous with the absorbent core (20).

4. A pantiliner according to any of the preceding claims wherein said core periphery (30) defines a generally oval shape.

5. A pantiliner according to claim 1 wherein the surface area of the fluid impermeable barrier layer (24) is smaller than the surface area of the absorbent core (20) and wherein the fluid impermeable barrier (24) is generally centered in the middle region (16) of the pantiliner (10).

6. A pantiliner according to any of the preceding claims wherein said fluid impermeable barrier (24) is a polyolefin film printed on at least one of its side with said coloured pattern (34).

7. A pantiliner according to any of the preceding claims wherein said pantiliner has a thickness of less than about 4 mm.

8. A pantiliner according to any of the preceding claims, wherein said topsheet and said backsheet have a common periphery (28).

9. A pantiliner according to any of the preceding claims wherein said topsheet and said core are joined by mechanical entangling of a portion of their respective fibers.

10. A pantiliner according to any of the preceding claims wherein the backsheet is made of a pigmented nonwoven material.
